(19) Europäisches Patentamt
European Patent Office
Office européen des brevets

(11) Veröffentlichungsnummer: **0 019 162**
**B1**

(12) # EUROPÄISCHE PATENTSCHRIFT

(45) Veröffentlichungstag der Patentschrift: **01.12.82**

(21) Anmeldenummer: **80102384.7**

(22) Anmeldetag: **02.05.80**

(51) Int. Cl.³: **C 12 P 33/06,** C 07 J 5/00, C 07 J 71/00

(54) **12-alpha-Hydroxysteroide und deren Herstellung.**

(30) Priorität: **15.05.79 DE 2919984**

(43) Veröffentlichungstag der Anmeldung: **26.11.80 Patentblatt 80/24**

(45) Bekanntmachung des Hinweises auf die Patenterteilung: **01.12.82 Patentblatt 82/48**

(84) Benannte Vertragsstaaten: **AT BE CH DE FR GB IT LI LU NL SE**

(56) Entgegenhaltungen:

**W. CHARNEY und H. L. HERZOG: «Microbial Transformations of Steroids», 1967, Seiten 338-339. Academic Press, New York und London. JOURNAL OF THE CHEMICAL SOCIETY, 1954, London, GB**

**W.J. ADAMS et al. «12-Oxygenated pregnane derivatives. Part I. (a) 21-acetoxy-12alpha: 17alpha-dihydroxypregn-4-ene-3:20-dione; (b) 12alpha: 21-di-acetoxy-17alpha-hydroxypregn-4-ene3:20-dione; (c) 3alpha 12alpha: 21-triacetoxy-17alpha-hydroxypreg-nan-20-one; and (d) miscellaneous observations», Seiten 1825-1836.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Kieslich, Klaus, Dr., Strasse zum Löwen 4, D-1000 Berlin 39 (DE)**
Erfinder: **Kerb, Ulrich, Dr., Prinzregentenstrasse 7, D-1000 Berlin 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# 12-alpha-Hydroxysteroide und deren Herstellung

Die Erfindung betrifft die in den Ansprüchen 2 und 3 gekennzeichneten 12α-Hydroxysteroide, sowie das im Anspruch 1 gekennzeichnete Verfahren zur Herstellung von 12α-Hydroxysteroiden.

Die bisher bekannten Verfahren zur mikrobiologischen Herstellung von 12α-Hydroxysteroiden sind technisch nicht anwendbar, da die hierbei erzielten Ausbeuten an gewünschtem Verfahrensprodukt zu gering sind. Es wurde nun gefunden, dass es mit Hilfe einer Kultur von Cercospora kaki (CBS 128 39) überraschenderweise möglich ist, Steroide der allgemeinen Formel II in relativ guten Ausbeuten in der 12α-Position zu hydroxylieren. Enthalten die Steroide der allgemeinen Formel II in der 21-Position als Substituenten $R_3$ eine Alkanoylgruppe — zum Beispiel eine Formylgruppe, eine Acetylgruppe, eine Propionylgruppe oder eine Butyrylgruppe — so wird diese bei der fermentativen Umwandlung verseift.

Das erfindungsgemässe Verfahren wird unter den Bedingungen durchgeführt, die man üblicherweise bei der mikrobiologischen Hydroxylierung von Steroiden anwendet.

Unter den üblicherweise verwendeten Kulturbedingungen werden in einem geeigneten Nährmedium unter Belüften, Submerskulturen angezüchtet. Dann setzt man den Kulturen das Substrat der allgemeinen Formel II (in einem Lösungsmittel gelöst oder in emulgierter Form) zu und fermentiert, bis eine maximale Substratumwandlung erreicht ist.

Geeignete Substratlösungsmittel sind beispielsweise Methanol, Äthanol, Glykolmomethyläther, Dimethylformamid, oder Dimethylsulfoxyd. Die Emulgierung des Substrates kann beispielsweise bewirkt werden, indem man diese in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxyaddukte oder Fettsäureester von Polyglykolen. Als geeignete Emulgatoren seien die handelsüblichen Netzmittel Tegin®, Tween® und Span beispielsmässig genannt.

Die optimale Substratkonzentration, Substratzugabezeit und Fermentationsdauer ist von den angewendeten Fermentationbedingungen abhängig. Diese Grössen müssen, wie dies bei mikrobiologischen Steroidumwandlungen allgemein erforderlich ist, im Einzelfall durch Vorversuche, wie sie dem Fachmann geläufig sind, ermittelt werden.

Die erhaltenen 12α-Hydroxysteroide der allgemeinen Formel I sind wertvolle Zwischenprodukte zur Herstellung pharmakologisch wirksamer Steroide. So kann man diese Verbindungen beispielsweise unter der gleichen Bedingungen wie die entsprechenden 11α- oder 11β-Hydroxysteroide in der 1,2-Position dehydrieren und dehydratisieren (US-Patent 3 232 839) und erhält die entsprechenden 1,4,11(12)-Pregnatrien-3,20-dion Derivate, welche in bekannter Weise in antiinflammatorisch wirksame 12-Halogen-11β-hydroxysteroide überführt werden können (Französisches Patent Nr. 1 506 008, US-Patent Nr. 3 053 837 und deutsche Offenlegungsschrift Nr. 26 32 678).

Die nachfolgenden Ausführungsbeispiele dienen zur Erläuterung des erfindungsgemässen Verfahrens.

## Beispiel 1

500 ml einer sterilen Nährlösung bestehend aus 10% flüssigem Stärkezucker, (50% Glucosegehalt), 0,5% Cornsteep liquor, 0,2% Natriumnitrat, 0,1% Kaliumdihydrogenphosphat, 0,05% Kaliumchlorid, 0,05% Magnesiumsulfatheptahydrat, 0,002% Eisen(II)-sulfatheptahydrat werden mit einer zwei Wochen alten Schrägagarkultur von Cercospora kaki (CBS 12 839) beimpft und fünf Tage lang bei 30°C geschüttelt. Die so erhaltene Vorkultur dient zur Beimpfung eines 20-l-Vorfermenters, der mit 15 l eines sterilen Mediums, enthaltend 10% flüssigen Stärkezucker, (50% Glucosegehalt), 0,5% Cornsteep liquor, 0,2% Natriumnitrat, 0,1% Kaliumdihydrogenphosphat, 0,05% Kaliumchlorid, 0,05% Magnesiumsulfatheptahydrat und 0,002% Eisen(II)-sulfatheptahydrat (mit 25% Natronlauge pH eingestellt auf 6,5) beschickt ist.

Man lässt die Vorkultur 48 bis 72 Stunden lang unter Rühren (220 U/Min.) und Belüftung (15 l Luft/Min.) bei 29°C anwachsen.

900 ml einer so erhaltenen Vorkultur werden in einen 20-l-Hauptfermenter überführt, welcher 14,1 l eines sterilen Mediums bestehend aus 6% flüssigem Stärkezucker, 1% Cornsteep liquor, 0,2% Natriumnitrat, 0,01% Kaliumdihydrogenphosphat, 0,2% Kaliumhydrogenphosphat, 0,05% Magnesiumsulfatheptahydrat, 0,002% Eisen(II)-sulfat und 0,05% Kaliumchlorid (pH eingestellt auf 5,8) beschickt ist. Man lässt die Kultur unter Rühren und Belüftung bei 29°C anwachsen und fügt nach 12 Stunden oder 24 Stunden eine sterilfiltrierte Lösung von 3,75 g 17α,21-Dihydroxy-4-pregnen-3,20-dion in 100 ml Dimethylformamid hinzu, wonach unter gleichen Bedingungen weiterfermentiert wird.

Die Kontrolle der Umsetzung erfolgt dünnschichtchromatographisch auf Fertigplatten Kieselgel 60 F 254 Merck von Methylisobutylketonextrakten von Intervallproben der Kulturbrühe.

Nach vollständiger Umwandlung des Ausgangsmaterials (ca. 37 Stunden) wird der Fermenteransatz filtriert, das Filtrat und das Mycel mit Methylisobutylketon extrahiert. Die Extrakte werden vereinigt und im Vakuum eingeengt.

Der erhaltene Rückstand wird zweimal mit je 100 ml Hexan von anhaftendem Antischaummittel (Silicon SH) gewaschen, über eine Kieselgelsäule chromatographiert und aus Essigester unter Zusatz von Aktivkohle umkristallisiert. Man erhält 1,16 g 12α,17α,21-Trihydroxy-4-pregnen-3,20-dion vom Schmelzpunkt 209-210°C.

Beispiel 2

Unter den Bedingungen von Beispiel 1 werden 1,2 g 17α,21-Dihydroxy-16α-methyl-4-pregnen-3,20-dion in 19 Stunden Kontaktzeit fermentiert. Man erhält 370 mg 12α,17α,21-Trihydroxy-16α-methyl-4-pregnen-3,20-dion vom Schmelzpunkt 237-239°C.

Beispiel 3

Unter den Bedingungen von Beispiel 1 werden 3,75 g 21-Hydroxy-6α-fluor-16α-methyl-4-pregnen-3,20-dion in 21 Stunden Kontaktzeit fermentiert. Man erhält nach chromatographischer Reinigung des Rohproduktes 895 mg 12α,21-Dihydroxy-6α-fluor-16α-methyl-4-pregnen-3,20-dion vom Schmelzpunkt 205-206°C.

**Patentansprüche**

1. Verfahren zur Herstellung von 12α-Hydroxysteroiden der allgemeinen Formel I

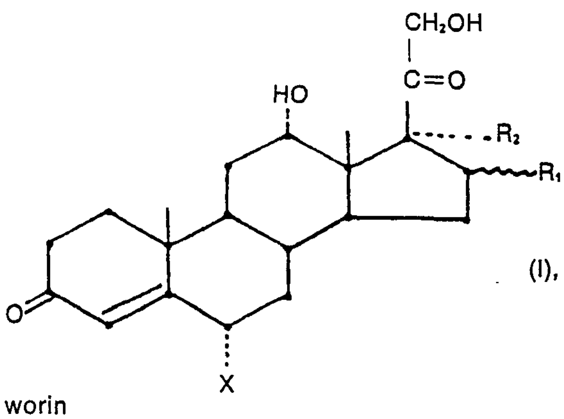

(I),

worin

X ein Wasserstoffatom oder ein Fluoratom bedeutet, und

$R_1$ ein Wasserstoffatom oder eine Methylgruppe sowie

$R_2$ ein Wasserstoffatom oder eine Hydroxygruppe oder

$R_1$ und $R_2$ gemeinsam eine Isopropylidendioxygruppe darstellen,

dadurch gekennzeichnet, dass man ein Steroid der allgemeinen Formel II

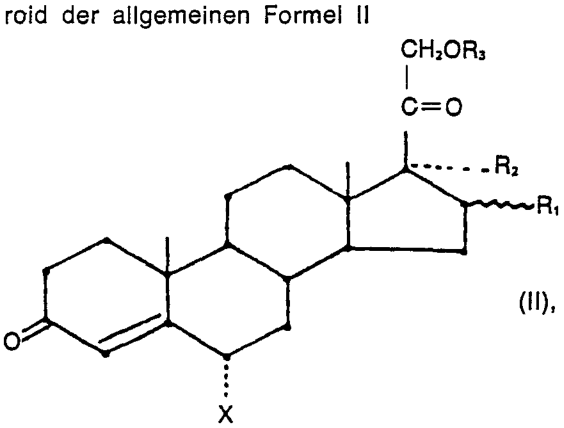

(II),

worin

X, $R_1$ und $R_2$ die obengenannte Bedeutung besitzen und

$R_3$ ein Wasserstoffatom oder eine Alkanoylgruppe mit 1 bis 6 Kohlenstoffatomen darstellt,

mit einer Kultur Cercospora kaki (CBS 128 39) fermentiert.

2. 12α,17α,21-Trihydroxy-16α-methyl-4-pregnen-3,20-dion.

3. 12α,21-Dihydroxy-6α-fluor-16α-methyl-4-pregnen-3,20-dion.

**Claims**

1. Process for the manufacture of 12α-hydroxysteroids of the general formula I

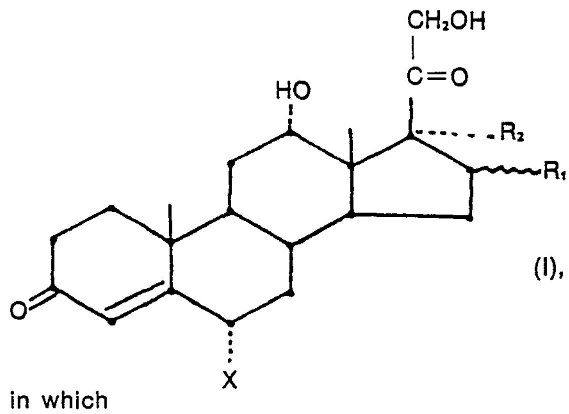

(I),

in which

X represents a hydrogen atom or a fluorine atom,

$R_1$ represents a hydrogen atom or a methyl group, and

$R_2$ represents a hydrogen atom or a hydroxy group, or

$R_1$ and $R_2$ together represent an isopropylidenedioxy group, characterised in that a steroid of the general formula II

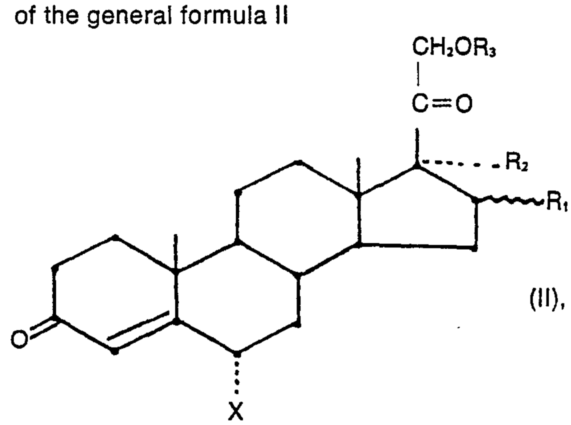

(II),

in which

X, $R_1$ and $R_2$ have the meanings given above and $R_3$ represents a hydrogen atom or an alkanoyl group having from 1 to 6 carbon atoms,

is fermented with a culture of Cercospora kaki (CBS 128 39).

2. 12α,17α,21-trihydroxy-4-pregnene-3,20-dione.

3. 12α,17α,21-trihydroxy-16α-methyl-4-pregnene-3,20-dione.

**Revendications**

1. Procédé pour la production de 12α-hydroxy-stéroïdes de formule générale I

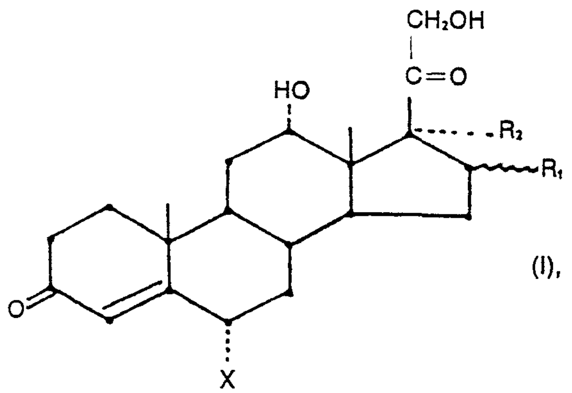

(I),

dans laquelle

X représente un atome d'hydrogène ou de fluor,

$R_1$ représente un atome d'hydrogène ou un radical méthyle, et

$R_2$ un atome d'hyrogène ou un radical hydroxyde ou $R_1$ et $R_2$ ensemble représentent un radical isopropylidène dioxy caractérisé en ce que l'on fait fermenter un stéroïde de formule générale II

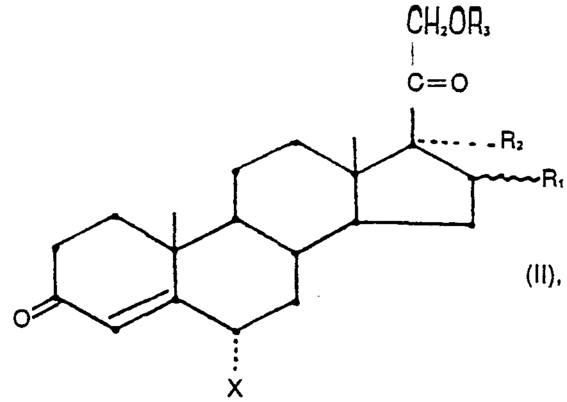

(II),

dans laquelle

X, $R_1$ et $R_2$ ont les précédentes significations et $R_3$ représente un atome d'hydrogène ou un radical alcanoyle de 1 à 6 atomes de carbone, avec une culture de Cercospora kaki (CBS 128 39).

2. La 12α,17α,21-trihydroxy-4-prégnène-3,20-dione.

3. La 12α,17α,21-trihydroxy-16α-méthyl-4-prégnène-3,20-dione.